# EUROPEAN PATENT APPLICATION

(11) **EP 4 249 597 A1**
(43) Date of publication of application: **27.09.2023**
(21) Application number: 21894729.9
(22) Date of filing: 18.11.2021
(51) Int. Cl.: C12N 15/54, A01H 5/00, A01H 6/14, A01H 6/56, A01H 6/74, A01H 6/82, C12N 5/04, C12N 5/10, C12N 9/10, C12N 15/53, C12N 15/63

(54) **FLAVONE 4'-O-METHYLTRANSFERASE GENE AND USE FOR SAME**

(30) Priority: 18.11.2020 JP 2020191753
(71) Applicant: Suntory Holdings Limited, Osaka 530-8203 (JP)
(72) Inventor: NAKAMURA, Noriko, Soraku-gun, Kyoto 619-0284 (JP)
(74) Representative: Vossius & Partner Patentanwälte Rechtsanwälte mbB
(86) International application number: PCT/JP2021/042477
(87) International publication number: WO 2022/107857

(57) **Abstract**

Transgenic plants with modified flower color, or their inbred or outbred progeny, or their propagules, partial plant bodies, tissues or cells, are provided. A delphinidin-type anthocyanin and a flavone C-glycoside in which the 7-position and 4'-position hydroxyl groups are methylated, are caused to coexist in plant cells.

## Description

### FIELD

The present invention relates to a novel polynucleotide encoding a protein having activity of transferring a methyl group to the 4'-position hydroxyl group of flavone C-glycoside, and to use of the same.

### BACKGROUND

Rose, petunia, chrysanthemum and carnation are industrially important ornamental plants worldwide. Rose in particular, being the most popular flowering plant, has a record of cultivation since ancient times, and it has been artificially crossbred for hundreds of years. One problem, however, has been that none of the hybridizable related species have wild varieties with blue flower color, and it has therefore been difficult to create rose varieties with blue flower color by conventional cross-breeding and mutation breeding. Creating completely new blue flower colors should lead to new demand for even wider uses of ornamental flowers, and should help to increase production and consumption. It has therefore been attempted to create roses with blue flower colors by genetic engineering methods.

Flowers with purple to blue colors, for example, are known to abundantly contain delphinidin-type anthocyanins having delphinidin, petunidin and malvidin backbones, but since ornamental flowers such as rose cannot produce such delphinidin-type anthocyanins, research continues to be conducted with the aim of artificially producing delphinidins by expressing the flavonoid 3',5'-hydroxylase gene that is necessary for their synthesis (NPL 1). However, even when plant metabolism is artificially modified in order to express an enzyme gene that produces a substance of interest in the recombinant plant, in many cases competition with endogenous enzymes of the same plant results in little or absolutely no accumulation of the substance of interest.

Moreover, the color of a flower changes not only by the structures of the anthocyanins themselves as the essential pigments, but also due to copresent flavonoids (also known as copigments), metal ions, and the vacuole pH. Flavones or flavonols are typical copigments that form sandwich-like layers with anthocyanins, thereby increasing blueness, deepening color and accentuating color tone (NPL 2). This is known as the "copigment effect". Flavones, in particular, are known to exhibit a powerful copigment effect, and analysis of gene recombinant carnations, for example, has demonstrated that flavones exhibit a significant copigment effect (NPL 3). For Dutch iris, it has been reported that a higher ratio of the total flavone content with respect to the total delphinidin content results in a more powerful copigment effect, and a bluer color (NPL 4). In Asiatic dayflower, blue coloration has been reported by formation of commelinin (a metal complex of malonyl awobanin, flavocommelin and magnesium ion) (NPL 9).

However, not all plants can produce flavones, and it is known that roses and petunias do not store flavones. Attempts have therefore been made to modify flower color by expressing in the plants different genes coding for proteins having activity for synthesizing flavones from flavanones (PTL 1).

In plants, flavones are distributed not only in free form but also as glycosides, with flavone O-glycosides and flavone C-glycosides being formed primarily, and flavone C-glycosides being known to exhibit a particularly powerful copigment effect. For example, isovitexin, as one type of flavone C-glycoside, has been reported to exhibit a copigment effect with anthocyanins in Japanese garden iris (*Iris ensata* Thunb.), and to stabilize a blue flower color by stabilizing anthocyanins (NPL 5). Two biosynthetic pathways have been reported so far for flavone C-glycosides, one being synthesis from flavanones by reaction catalyzed by flavanone 2-hydroxylase, flavone C-glycosyltransferase and dehydratase. Another is synthesis from flavanones by reaction catalyzed by flavone synthase and flavone C-glycosyltransferase (NPL 6).

The copigment effect is also thought to depend on the quantity ratio of anthocyanins and flavones, as well as sugar and methyl or acyl group modification of the anthocyanins and flavones, and therefore blue flower color cannot necessarily be obtained simply by expressing a flavone synthase gene and causing accumulation of flavones. When the torenia flavone synthase gene is expressed in petunia, the violet flower color is fainter (NPL 7). Moreover, expression of the gentian-derived flavone synthase gene in tobacco results in flavone synthesis (NPL 8), but similarly results in a fainter flower color. The flavone C-glycosyltransferase contributing to biosynthesis of isosaponarins in wasabi has been identified (NPL 12) and it has also been attempted to artificially add flavones and malvidins to modify rose flower color (PTL 2), but no success has yet been achieved in creating roses with blue flower color.

In recent years, the present inventors have obtained a flavone 7-O-methyltransferase gene that transfers a methyl group to the 7-position hydroxyl group of flavone C-glycoside from oboushibana (*Commelina communis* var. hortensis), which is known as a (cultivated) variant of Asiatic dayflower, and we have used it to successfully obtain a rose with bluer flower color than the prior art, by causing swertisin and a delphinidin-type anthocyan to coexist in the plant petals (PTL 3). However a need still exists for development of blue color expression-regulating technology allowing creation of roses having truly blue color.

### [CITATION LIST]

### [PATENT LITERATURE]

[PTL 1] Japanese Unexamined Patent Publication No. 2000-279182
[PTL 2] International Patent Publication No. WO2008/156206
[PTL 3] International Patent Publication No. WO2020/203940

### [NON PATENT LITERATURE]

[NPL 1] Phytochemistry Reviews 5,283-291
[NPL 2] Prog. Chem. Org. Natl. Prod.52
[NPL 3] Phytochemistry, 63,15-23(2003)
[NPL 4] Plant Physiol. Bioch. 72, 116-124(2013)
[NPL 5] Euphytica 115, 1-5(2000)
[NPL 6] FEBS Lett. 589, 182-187(2015)
[NPL 7] Plant Biotechnology, 21, 377-386(2004)
[NPL 8] Molecular Breeding 17:91-99(2006)
[NPL 9] Proceedings of the Japan Academy. Ser. B: Physical and Biological Sciences 84(10), 452-456, 2008
[NPL 10] Plant Mol. Biol. 36(2), 219-227(1998)
[NPL 11] Plant Mol. Biol. 62(4-5), 715-733(2006)
[NPL 12] Plant Cell Physiol. 60(12):2733-2743(2019)

### SUMMARY

### [TECHNICAL PROBLEM]

The problem to be solved by the present invention is to provide a transgenic plant with modified flower color, or its inbred or outbred progeny, or their propagules, partial plant bodies, tissues or cells.

### [SOLUTION TO PROBLEM]

As a result of much ardent research and experimentation, the present inventors have found that if a delphinidin-type anthocyanin and a flavone C-glycoside are caused to coexist in the petals of a plant, it is possible to obtain a transgenic plant having flower color that has not been obtainable in the prior art, and specifically a rose plant having blue flower color (Violet-Blue group/Blue group according to RHS Color Chart 5th Edition, and/or hue angle: 339.7° to 270.0°). Even more surprisingly, the present inventors found that, among numerous flavone C-glycosides, the combination with embigenin, wherein not only the 7-position hydroxyl group but also the 4'-position hydroxyl group is methylated, results in a bluer color, and have succeeded in obtaining a novel flavone 4'-O-methyltransferase gene that transfers a methyl group to the 4'-position hydroxyl group of flavone C-glycoside, from fringed iris (*Iris japonica*)*.* The invention has been completed based on these findings.

Specifically, the present invention provides the following.
[1] A polynucleotide selected from the group consisting of the following (A) to (E):
   (A) a polynucleotide comprising the nucleotide sequence of SEQ ID NO: 19 or SEQ ID NO: 21;
   (B) a polynucleotide that hybridizes with a polynucleotide comprising the nucleotide sequence complementary to the nucleotide sequence of SEQ ID NO: 19 or SEQ ID NO: 21 under stringent conditions, and that encodes a protein having activity of transferring a methyl group to the 4'-position hydroxyl group of flavone C-glycoside;
   (C) a polynucleotide encoding a protein comprising the amino acid sequence of SEQ ID NO: 20;
   (D) a polynucleotide encoding a protein comprising an amino acid sequence that is the amino acid sequence of SEQ ID NO: 20 having a deletion, substitution, insertion and/or addition of one or more amino acids, and having activity of transferring a methyl group to the 4'-position hydroxyl group of flavone C-glycoside; and
   (E) a polynucleotide encoding a protein comprising an amino acid sequence with at least 90% identity with respect to the amino acid sequence of SEQ ID NO: 20 and having activity of transferring a methyl group to the 4'-position hydroxyl group of flavone C-glycoside.
[2] The polynucleotide according to [1], which is a polynucleotide comprising the nucleotide sequence of SEQ ID NO: 19 or SEQ ID NO: 21.
[3] The polynucleotide according to [1], which is a polynucleotide encoding a protein comprising the amino acid sequence of SEQ ID NO: 20.
[4] A protein encoded by a polynucleotide according to any one of [1] to [3].
[5] A vector comprising a polynucleotide according to any one of [1] to [3].
[6] A vector according to [5], further comprising a flavone 7-O-methyltransferase (Fn-7OMT) gene or its homolog.
[7] A vector according to [6], wherein the Fn-7OMT gene or its homolog is selected from the group consisting of:
   (a) a polynucleotide comprising the nucleotide sequence of SEQ ID NO: 1;
   (b) a polynucleotide that hybridizes with a polynucleotide comprising the nucleotide sequence complementary to the nucleotide sequence of SEQ ID NO: 1 under stringent conditions, and that encodes a protein having activity of transferring a methyl group to the 7-position hydroxyl group of flavone C-glycoside;
   (c) a polynucleotide encoding a protein comprising the amino acid sequence of SEQ ID NO: 2;
   (d) a polynucleotide encoding a protein comprising an amino acid sequence that is the amino acid sequence of SEQ ID NO: 2 having a deletion, substitution, insertion and/or addition of one or more amino acids, and having activity of transferring a methyl group to the 7-position hydroxyl group of flavone C-glycoside; and
   (e) a polynucleotide encoding a protein comprising an amino acid sequence with at least 90% identity with respect to the amino acid sequence of SEQ ID NO: 2 and having activity of transferring a methyl group to the 7-position hydroxyl group of flavone C-glycoside.
[8] A vector according to any one of [5] to [7], further comprising a flavone synthase (FNS) gene or its homolog, and a flavone C-glycosyltransferase (CGT) gene or its homolog.
[9] A vector according to [8], wherein
   the FNS gene or its homolog is selected from the group consisting of:
      (1-a) a polynucleotide comprising the nucleotide sequence of SEQ ID NO: 3;
      (1-b) a polynucleotide that hybridizes with a polynucleotide comprising the nucleotide sequence complementary to the nucleotide sequence of SEQ ID NO: 3 under stringent conditions and encodes a protein having the same activity as a protein encoded by the polynucleotide of (1-a);
      (1-c) a polynucleotide encoding a protein comprising the amino acid sequence of SEQ ID NO: 4;
      (1-d) a polynucleotide encoding a protein comprising an amino acid sequence that is the amino acid sequence of SEQ ID NO: 4 having a deletion, substitution, insertion and/or addition of one or more amino acids, and having the same activity as a protein encoded by the polynucleotide of (1-c); and
      (1-e) a polynucleotide encoding a protein having an amino acid sequence with at least 90% identity with respect to the amino acid sequence of SEQ ID NO: 4 and having the same activity as a protein encoded by the polynucleotide of (1-c), and
   the flavone CGT gene or its homolog is selected from the group consisting of:
      (2-a) a polynucleotide comprising the nucleotide sequence of SEQ ID NO: 5 or SEQ ID NO: 22;
      (2-b) a polynucleotide that hybridizes with a polynucleotide comprising the nucleotide sequence complementary to the nucleotide sequence of SEQ ID NO: 5 or SEQ ID NO: 22 under stringent conditions and encodes a protein having the same activity as a protein encoded by the polynucleotide of (2-a);
      (2-c) a polynucleotide encoding a protein comprising the amino acid sequence of SEQ ID NO: 6 or SEQ ID NO: 23;
      (2-d) a polynucleotide encoding a protein comprising an amino acid sequence that is the amino acid sequence of SEQ ID NO: 6 or SEQ ID NO: 23 having a deletion, substitution, insertion and/or addition of one or more amino acids, and having the same activity as a protein encoded by the polynucleotide of (2-c); and
      (2-e) a polynucleotide encoding a protein having an amino acid sequence with at least 90% identity with respect to the amino acid sequence of SEQ ID NO: 6 or SEQ ID NO: 23 and having the same activity as a protein encoded by the polynucleotide of (2-c).
[10] A vector according to [8] or [9], further comprising a flavonoid 3',5'-hydroxylase (F3'5'H) gene or its homolog, and a methyltransferase (MT) gene or its homolog.
[11] A vector according to [10], wherein
   the F3'5'H gene or its homolog is selected from the group consisting of:
      (3-a) a polynucleotide comprising the nucleotide sequence of SEQ ID NO: 7;
      (3-b) a polynucleotide that hybridizes with a polynucleotide comprising the nucleotide sequence complementary to the nucleotide sequence of SEQ ID NO: 7 under stringent conditions and encodes a protein having the same activity as a protein encoded by the polynucleotide of (3-a);
      (3-c) a polynucleotide encoding a protein comprising the amino acid sequence of SEQ ID NO: 8;
      (3-d) a polynucleotide encoding a protein comprising an amino acid sequence that is the amino acid sequence of SEQ ID NO: 8 having a deletion, substitution, insertion and/or addition of one or more amino acids, and having the same activity as a protein encoded by the polynucleotide of (3-c); and
      (3-e) a polynucleotide encoding a protein having an amino acid sequence with at least 90% identity with respect to the amino acid sequence of SEQ ID NO: 8 and having the same activity as a protein encoded by the polynucleotide of (3-c), and
   the MT gene or its homolog is selected from the group consisting of:
      (4-a) a polynucleotide comprising the nucleotide sequence of SEQ ID NO: 9;
      (4-b) a polynucleotide that hybridizes with a polynucleotide comprising the nucleotide sequence complementary to the nucleotide sequence of SEQ ID NO: 9 under stringent conditions and encodes a protein having the same activity as a protein encoded by the polynucleotide of (4-a);
      (4-c) a polynucleotide encoding a protein comprising the amino acid sequence of SEQ ID NO: 10;
      (4-d) a polynucleotide encoding a protein comprising an amino acid sequence that is the amino acid sequence of SEQ ID NO: 10 having a deletion, substitution, insertion and/or addition of one or more amino acids, and having the same activity as a protein encoded by the polynucleotide of (4-c); and
      (4-e) a polynucleotide encoding a protein having an amino acid sequence with at least 90% identity with respect to the amino acid sequence of SEQ ID NO: 10 and having the same activity as a protein encoded by the polynucleotide of (4-c).
[12] A vector according to any one of [9] to [11], wherein *Arabidopsis thaliana* alcohol dehydrogenase (ADH) gene 5'-untranslated region (5'-UTR) (SEQ ID NO: 11) is attached to the flavone CGT gene or its homolog.
[13] A vector according to any one of [5] to [7], further comprising the flavanone 2-hydroxylase (F2H) gene or its homolog, the flavone C-glycosyltransferase (CGT) gene or its homolog, and the dehydratase (FDH) gene or its homolog.
[14] A vector according to [13], further comprising the flavonoid F3'5' hydroxylase (F3'5'H) gene or its homolog, and the methyltransferase (MT) gene or its homolog.
[15] A vector according to 14, wherein
   the F2H gene or its homolog is selected from the group consisting of:
      (5-a) a polynucleotide comprising the nucleotide sequence of SEQ ID NO: 12;
      (5-b) a polynucleotide that hybridizes with a polynucleotide comprising the nucleotide sequence complementary to the nucleotide sequence of SEQ ID NO: 12 under stringent conditions and encodes a protein having the same activity as a protein encoded by the polynucleotide of (5-a);
      (5-c) a polynucleotide encoding a protein comprising the amino acid sequence of SEQ ID NO: 13;
      (5-d) a polynucleotide encoding a protein comprising an amino acid sequence that is the amino acid sequence of SEQ ID NO: 13 having a deletion, substitution, insertion and/or addition of one or more amino acids, and having the same activity as a protein encoded by the polynucleotide of (5-c); and
      (5-e) a polynucleotide encoding a protein having an amino acid sequence with at least 90% identity with respect to the amino acid sequence of SEQ ID NO: 13 and having the same activity as a protein encoded by the polynucleotide of (5-c),
   the flavone CGT gene or its homolog is selected from the group consisting of:
      (6-a) a polynucleotide comprising the nucleotide sequence of SEQ ID NO: 16;
      (6-b) a polynucleotide that hybridizes with a polynucleotide comprising the nucleotide sequence complementary to the nucleotide sequence of SEQ ID NO: 16 under stringent conditions and encodes a protein having the same activity as a protein encoded by the polynucleotide of (6-a);
      (6-c) a polynucleotide encoding a protein comprising the amino acid sequence of SEQ ID NO: 17;
      (6-d) a polynucleotide encoding a protein comprising an amino acid sequence that is the amino acid sequence of SEQ ID NO: 17 having a deletion, substitution, insertion and/or addition of one or more amino acids, and having the same activity as a protein encoded by the polynucleotide of (6-c); and
      (6-e) a polynucleotide encoding a protein having an amino acid sequence with at least 90% identity with respect to the amino acid sequence of SEQ ID NO: 17 and having the same activity as a protein encoded by the polynucleotide of (6-c),
   the FDH gene or its homolog is selected from the group consisting of:
      (7-a) a polynucleotide comprising the nucleotide sequence of SEQ ID NO: 14;
      (7-b) a polynucleotide that hybridizes with a polynucleotide comprising the nucleotide sequence complementary to the nucleotide sequence of SEQ ID NO: 14 under stringent conditions and encodes a protein having the same activity as a protein encoded by the polynucleotide of (7-a);
      (7-c) a polynucleotide encoding a protein comprising the amino acid sequence of SEQ ID NO: 15;
      (7-d) a polynucleotide encoding a protein comprising an amino acid sequence that is the amino acid sequence of SEQ ID NO: 15 having a deletion, substitution, insertion and/or addition of one or more amino acids, and having the same activity as a protein encoded by the polynucleotide of (7-c); and
      (7-e) a polynucleotide encoding a protein having an amino acid sequence with at least 90% identity with respect to the amino acid sequence of SEQ ID NO: 15 and having the same activity as a protein encoded by the polynucleotide of (7-c),
   the F3'5'H gene or its homolog is selected from the group consisting of:
      (8-a) a polynucleotide comprising the nucleotide sequence of SEQ ID NO: 7;
      (8-b) a polynucleotide that hybridizes with a polynucleotide comprising the nucleotide sequence complementary to the nucleotide sequence of SEQ ID NO: 7 under stringent conditions and encodes a protein having the same activity as a protein encoded by the polynucleotide of (8-a);
      (8-c) a polynucleotide encoding a protein comprising the amino acid sequence of SEQ ID NO: 8;
      (8-d) a polynucleotide encoding a protein comprising an amino acid sequence that is the amino acid sequence of SEQ ID NO: 8 having a deletion, substitution, insertion and/or addition of one or more amino acids, and having the same activity as a protein encoded by the polynucleotide of (8-c); and
      (8-e) a polynucleotide encoding a protein having an amino acid sequence with at least 90% identity with respect to the amino acid sequence of SEQ ID NO: 8 and having the same activity as a protein encoded by the polynucleotide of (8-c), and
   the MT gene or its homolog is selected from the group consisting of:
      (9-a) a polynucleotide comprising the nucleotide sequence of SEQ ID NO: 9;
      (9-b) a polynucleotide that hybridizes with a polynucleotide comprising the nucleotide sequence complementary to the nucleotide sequence of SEQ ID NO: 9 under stringent conditions and encodes a protein having the same activity as a protein encoded by the polynucleotide of (9-a);
      (9-c) a polynucleotide encoding a protein comprising the amino acid sequence of SEQ ID NO: 10;
      (9-d) a polynucleotide encoding a protein comprising an amino acid sequence that is the amino acid sequence of SEQ ID NO: 10 having a deletion, substitution, insertion and/or addition of one or more amino acids, and having the same activity as a protein encoded by the polynucleotide of (9-c); and
      (9-e) a polynucleotide encoding a protein having an amino acid sequence with at least 90% identity with respect to the amino acid sequence of SEQ ID NO: 10 and having the same activity as a protein encoded by the polynucleotide of (9-c).
[16] A vector according to [15], wherein *Arabidopsis thaliana* HSPRO gene untranslated region (5'-UTR) (SEQ ID NO: 18) is attached to the flavone CGT gene.
[17] A transgenic plant, or its inbred or outbred progeny, comprising a polynucleotide according to any one of [1] to [3].
[18] The transgenic plant according to [17], or its inbred or outbred progeny, wherein the plant is selected from among a rose, petunia, chrysanthemum, carnation or lily.
[19] The transgenic plant according to [18], or its inbred or outbred progeny, wherein the plant is a rose.
[20] Propagules, partial plant bodies, tissue or cells of a transgenic plant according to any one of [17] to [19] or its inbred or outbred progeny.
[21] Cut flowers of a transgenic plant according to any one of [17] to [19], or its inbred or outbred progeny, or a processed form created from the cut flowers.
[22] A method for creating a transgenic plant with modified flower color, wherein the method comprises a step of causing a delphinidin-type anthocyanin and a flavone C-glycoside to coexist in plant cells, where the 7-position and 4'-position hydroxyl groups of flavone C-glycoside are methylated.
[23] The method according to [22], wherein the flavone C-glycoside is embigenin.
[24] The method according to [22] or [23], wherein the delphinidin-type anthocyanin is selected from the group consisting of malvidin 3,5-diglucoside (malvin), delphinidin 3,5-diglucoside (delphin), petunidin 3,5-diglucoside, acylated delphin and acylated malvin, and their combinations.
[25] The method according to any one of [22] to [24], comprising a step of introducing a vector according to any one of [5] to [16] into plant cells.
[26] The method according to [25], wherein the plant is a rose, petunia, chrysanthemum, carnation or lily.
[27] The method according to [26], wherein the plant is a rose.

### [ADVANTAGEOUS EFFECTS OF INVENTION]

According to the invention it is possible to create plant varieties with flower colors that have not been obtainable in the prior art.

### BRIEF DESCRIPTION OF DRAWINGS

Fig. 1 shows the biosynthetic pathway for a flavone C-glycoside in a plant.
Fig. 2 shows high-performance liquid chromatograms of a crude protein solution extract from *E. coli* expressing DN144, and of an enzyme reaction solution obtained by enzyme reaction on isovitexin.
Fig. 3 shows high-performance liquid chromatograms of a crude protein solution extract from *E. coli* expressing DN144, and of an enzyme reaction solution obtained by enzyme reaction on swertisin.

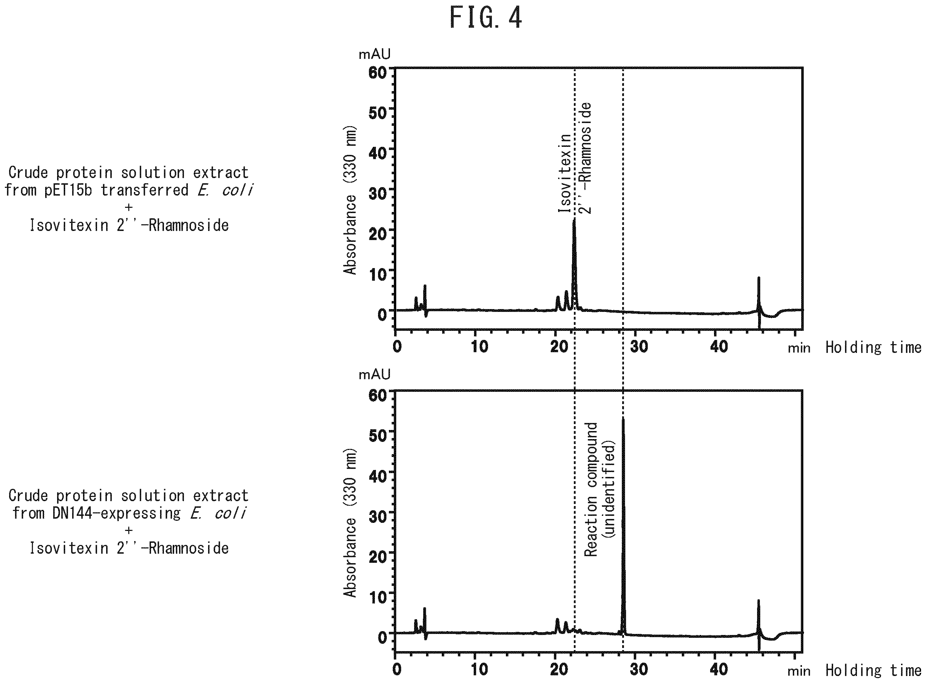

Fig. 4 shows high-performance liquid chromatograms of a crude protein solution extract from *E. coli* expressing DN144, and of an enzyme reaction solution obtained by enzyme reaction on isovitexin 2"-rhamnoside.
Fig. 5 is a structural diagram of binary vector pSPB7993 (top) and binary vector pSPB7994 (bottom).

Anthocyanins are a group of pigments that are widely extant in plants, and they are known to exhibit red, blue and purple flower colors. They are classified into 3 types, pelargonidin, cyanidin and delphinidin, based on the number of hydroxyl groups on the B-ring of the anthocyanidin, as the aglycone form. The chromophoric group is the aglycone portion, with pelargonidin-type anthocyanins exhibiting orange color, cyanidin-type anthocyanins exhibiting red color and delphinidin-type anthocyanins exhibiting purple to blue color. For the purpose of the present specification, "delphinidin-type anthocyanins" also include their derivatives having delphinidin, malvidin or petunidin backbones, with malvidin being preferred.

When delphinidin-type anthocyanins are copresent with substances such as flavones, flavonols, organic acid esters and tannins, their molecular interaction often produces blueish colors. This phenomenon is known as "copigmentation", and substances that produce the phenomenon are known as copigments. Copigmentation includes not only a color depth effect that induces blue color production, but also a deep color effect or an effect of increasing color stability. The present inventors have confirmed that copigmentation between delphinidin-type anthocyanins and flavone C-glycosides causes blue color expression in rose petals.

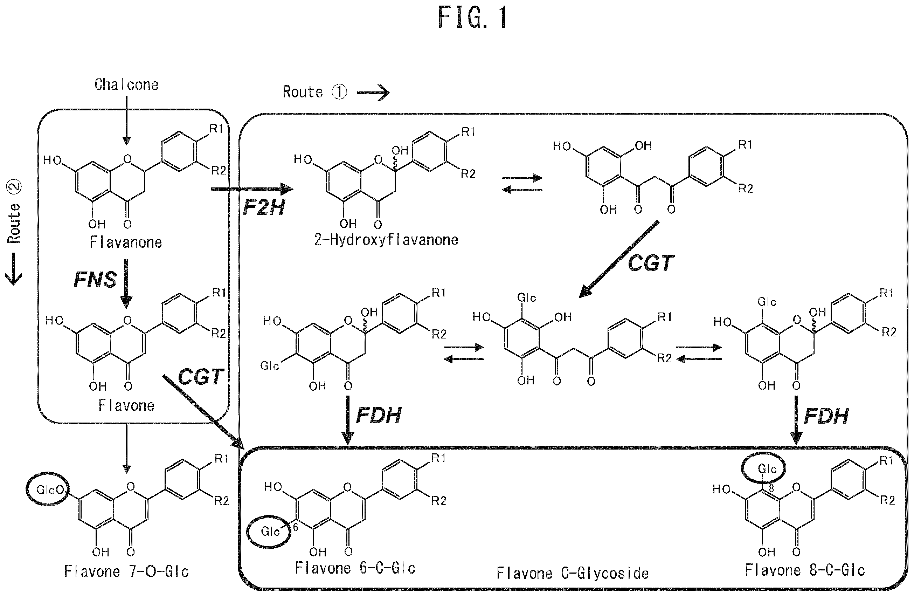

Flavones are organic compounds that are flavan-derived cyclic ketones, and in plants they mainly exist as glycosides. Flavone, in the strict definition, refers to 2,3-didehydroflavan-4-one, which is a compound with chemical formula C₁₅H₁₀O₂ and molecular weight 222.24, but in the wider sense flavones are a category of flavonoids, a flavonoid being classified as a "flavone" if it has a flavone structure as the basic backbone and also lacks the hydroxyl group at the 3-position. As used herein, "flavone C-glycoside" means a glycoside of a flavone in the wide sense, i.e. a derivative falling under the definition of flavones, wherein an aglycone is directly bonded to the anomeric carbon of an aldose. Flavone C-glycosides include, but are not limited to, luteolin C-glycoside, tricetin C-glycoside, apigenin C-glycoside and acacetin C-glycoside. Flavone C-glycosides also include glycosides of apigenin, luteolin, tricetin and acacetin derivatives. Two routes are known for the biosynthetic pathway of flavone C-glycosides in plants (Fig. 1). In route 1, a flavone 6-C-glucoside and flavone 8-C-glucoside are produced through the activity of flavanone 2-hydroxylase (F2H), flavone C-glycosyltransferase (CGT) and dehydratase (FDH). In route 2, on the other hand, flavone 6-C-glucoside is produced through the activity of flavone synthase (FNS) and flavone C-glycosyltransferase (CGT). The flavone C-glycoside is preferably selected from the group consisting of flavone 6-C-glucosides, flavone 8-C-glucosides and their combinations, examples of which include apigenin 6-C-glucoside (isovitexin), apigenin 8-C-glucoside (vitexin), luteolin 6-C-glucoside (isoorientin), luteolin 8-C-glucoside (orientin), tricetin 6-C-glucoside, tricetin 8-C-glucoside, and their derivatives.

Accumulation of flavone C-glycosides in plant cells can be achieved by transformation of a host plant with a vector comprising genes necessary for route 1 (i.e. the flavanone 2-hydroxylase (F2H) gene, flavone C-glycosyltransferase (CGT) gene and dehydratase (FDH) gene) or their homologs, or a vector comprising genes necessary for route 2 (i.e. the flavone synthase (FNS) gene and flavone C-glycosyltransferase (CGT) gene) or their homologs.

The source of the F2H gene or its homolog as a gene necessary for route 1 is not particularly restricted so long as it has the desired function, but it is preferably a licorice-derived F2H gene or its homolog, and selected from the group consisting of the following polynucleotides:
(a) a polynucleotide comprising the nucleotide sequence of SEQ ID NO: 12;
(b) a polynucleotide that hybridizes with a polynucleotide comprising the nucleotide sequence complementary to the nucleotide sequence of SEQ ID NO: 12 under stringent conditions and encodes a protein having the same activity as a protein encoded by the polynucleotide of (a);
(c) a polynucleotide encoding a protein comprising the amino acid sequence of SEQ ID NO: 13;
(d) a polynucleotide encoding a protein comprising an amino acid sequence that is the amino acid sequence of SEQ ID NO: 13 having a deletion, substitution, insertion and/or addition of one or more amino acids, and having the same activity as a protein encoded by the polynucleotide of (c); and
(e) a polynucleotide encoding a protein having an amino acid sequence with at least 90% identity with respect to the amino acid sequence of SEQ ID NO: 13 and having the same activity as a protein encoded by the polynucleotide of (c).

The source of the flavone CGT gene or its homolog as a gene necessary for route 1 is not particularly restricted so long as it has the desired function, but it is preferably a buckwheat-derived codon usage-modified flavone CGT gene or its homolog, and selected from the group consisting of the following polynucleotides:
(a) a polynucleotide comprising the nucleotide sequence of SEQ ID NO: 16;
(b) a polynucleotide that hybridizes with a polynucleotide comprising the nucleotide sequence complementary to the nucleotide sequence of SEQ ID NO: 16 under stringent conditions and encodes a protein having the same activity as a protein encoded by the polynucleotide of (a);
(c) a polynucleotide encoding a protein comprising the amino acid sequence of SEQ ID NO: 17;
(d) a polynucleotide encoding a protein comprising an amino acid sequence that is the amino acid sequence of SEQ ID NO: 17 having a deletion, substitution, insertion and/or addition of one or more amino acids, and having the same activity as a protein encoded by the polynucleotide of (c); and
(e) a polynucleotide encoding a protein having an amino acid sequence with at least 90% identity with respect to the amino acid sequence of SEQ ID NO: 17 and having the same activity as a protein encoded by the polynucleotide of (c).

The source of the FDH gene or its homolog as a gene necessary for route 1 is not particularly restricted so long as it has the desired function, but it is preferably a *Lotus japonicus-*derived FDH gene or its homolog, and selected from the group consisting of the following polynucleotides:
(a) a polynucleotide comprising the nucleotide sequence of SEQ ID NO: 14;
(b) a polynucleotide that hybridizes with a polynucleotide comprising the nucleotide sequence complementary to the nucleotide sequence of SEQ ID NO: 14 under stringent conditions and encodes a protein having the same activity as a protein encoded by the polynucleotide of (a);
(c) a polynucleotide encoding a protein comprising the amino acid sequence of SEQ ID NO: 15;
(d) a polynucleotide encoding a protein comprising an amino acid sequence that is the amino acid sequence of SEQ ID NO: 15 having a deletion, substitution, insertion and/or addition of one or more amino acids, and having the same activity as a protein encoded by the polynucleotide of (c); and
(e) a polynucleotide encoding a protein having an amino acid sequence with at least 90% identity with respect to the amino acid sequence of SEQ ID NO: 15 and having the same activity as a protein encoded by the polynucleotide of (c).

The source of the FNS gene or its homolog as a gene necessary for route 2 is not particularly restricted so long as it has the desired function, but it is preferably a torenia-derived FNS gene or its homolog, and selected from the group consisting of:
(a) a polynucleotide comprising the nucleotide sequence of SEQ ID NO: 3;
(b) a polynucleotide that hybridizes with a polynucleotide comprising the nucleotide sequence complementary to the nucleotide sequence of SEQ ID NO: 3 under stringent conditions and encodes a protein having the same activity as a protein encoded by the polynucleotide of (a);
(c) a polynucleotide encoding a protein comprising the amino acid sequence of SEQ ID NO: 4;
(d) a polynucleotide encoding a protein comprising an amino acid sequence that is the amino acid sequence of SEQ ID NO: 4 having a deletion, substitution, insertion and/or addition of one or more amino acids, and having the same activity as a protein encoded by the polynucleotide of (c); and
(e) a polynucleotide encoding a protein having an amino acid sequence with at least 90% identity with respect to the amino acid sequence of SEQ ID NO: 4 and having the same activity as a protein encoded by the polynucleotide of (c).

The source of the flavone CGT gene or its homolog as a gene necessary for route 2 is not particularly restricted so long as it has the desired function, but it is preferably a gentian-derived or wasabi-derived flavone CGT gene or its homolog, and selected from the group consisting of:
(a) a polynucleotide comprising the nucleotide sequence of SEQ ID NO: 5 or SEQ ID NO: 22;
(b) a polynucleotide that hybridizes with a polynucleotide comprising the nucleotide sequence complementary to the nucleotide sequence of SEQ ID NO: 5 or SEQ ID NO: 22 under stringent conditions and encodes a protein having the same activity as a protein encoded by the polynucleotide of (a);
(c) a polynucleotide encoding a protein comprising the amino acid sequence of SEQ ID NO: 6 or SEQ ID NO: 23;
(d) a polynucleotide encoding a protein comprising an amino acid sequence that is the amino acid sequence of SEQ ID NO: 6 or SEQ ID NO: 23 having a deletion, substitution, insertion and/or addition of one or more amino acids, and having the same activity as a protein encoded by the polynucleotide of (c); and
(e) a polynucleotide encoding a protein having an amino acid sequence with at least 90% identity with respect to the amino acid sequence of SEQ ID NO: 6 or SEQ ID NO: 23 and having the same activity as a protein encoded by the polynucleotide of (c).

A flavone CGT gene or its homolog as a gene necessary for route 2 preferably has *Arabidopsis thaliana* alcohol dehydrogenase (ADH) gene 5'-untranslated region (5'-UTR) (SEQ ID NO: 11) attached.

The present inventors found that, among flavone C-glycosides, the combination with swertisin, as a flavone C-glycoside wherein the 7-position hydroxyl group is methylated, results in a bluer color, and have already succeeded in obtaining the flavone 7-O-methyltransferase gene that transfers a methyl group to the 7-position hydroxyl group of a flavone C-glycoside obtained by route 1 or 2, from oboushibana (*Commelina communis* var. hortensis), as a known (cultivated) variant of Asiatic dayflower (PTL 3).

An oboushibana-derived flavone 7-O-methyltransferase (CcFn-7OMT) gene or its homolog is selected from the group consisting of the following (a) to (e):
(a) a polynucleotide comprising the nucleotide sequence of SEQ ID NO: 1;
(b) a polynucleotide that hybridizes with a polynucleotide comprising the nucleotide sequence complementary to the nucleotide sequence of SEQ ID NO: 1 under stringent conditions and encodes a protein having activity of transferring a methyl group to the 7-position hydroxyl group of flavone C-glycoside;
(c) a polynucleotide encoding a protein comprising the amino acid sequence of SEQ ID NO: 2;
(d) a polynucleotide encoding a protein comprising an amino acid sequence that is the amino acid sequence of SEQ ID NO: 2 having a deletion, substitution, insertion and/or addition of one or more amino acids, and having activity of transferring a methyl group to the 7-position hydroxyl group of flavone C-glycoside; and
(e) a polynucleotide encoding a protein having an amino acid sequence with at least 90% identity with respect to the amino acid sequence of SEQ ID NO: 2 and having activity of transferring a methyl group to the 7-position hydroxyl group of flavone C-glycoside.

In current research, the present inventors have found, surprisingly, that among flavone C-glycosides, the combination with embigenin, as a flavone C-glycoside wherein not only the 7-position hydroxyl group but also the 4'-position hydroxyl group is methylated, results in a bluer color, and have succeeded in obtaining a novel flavone 4'-O-methyltransferase gene that transfers a methyl group to 4'-position hydroxyl group of flavone C-glycoside obtained by route 1 or 2, from fringed iris.

A fringed iris-derived flavone 4'-O-methyltransferase (IjFn-4'OMT) gene or its homolog is selected from the group consisting of the following (A) to (E):
(A) a polynucleotide comprising the nucleotide sequence of SEQ ID NO: 19 or SEQ ID NO: 21;
(B) a polynucleotide that hybridizes with a polynucleotide comprising the nucleotide sequence complementary to the nucleotide sequence of SEQ ID NO: 19 or SEQ ID NO: 21 under stringent conditions and encodes a protein having activity of transferring a methyl group to the 4'-position hydroxyl group of flavone C-glycoside;
(C) a polynucleotide encoding a protein comprising the amino acid sequence of SEQ ID NO: 20;
(D) a polynucleotide encoding a protein comprising an amino acid sequence that is the amino acid sequence of SEQ ID NO: 20 having a deletion, substitution, insertion and/or addition of one or more amino acids, and having activity of transferring a methyl group to the 4'-position hydroxyl group of flavone C-glycoside; and
(E) a polynucleotide encoding a protein having an amino acid sequence with at least 90% identity with respect to the amino acid sequence of SEQ ID NO: 20 and having activity of transferring a methyl group to the 4'-position hydroxyl group of flavone C-glycoside.

Accumulation of delphinidin-type anthocyanins in plant cells can be achieved by incorporating a flavonoid 3',5'-hydroxylase (F3'5'H) gene or its homolog and a methyltransferase (MT) gene or its homolog in a host plant (PTL 2). By transforming a host plant with a vector comprising a F3'5'H gene or its homolog and an MT gene or its homolog in addition to a gene necessary for route 1 or its homolog, or a gene necessary for route 2 or its homolog, it is possible to cause a delphinidin-type anthocyanin and a flavone C-glycoside to coexist in the host plant cells.

The source of the F3'5'H gene or its homolog is not particularly restricted so long as it has the desired function, but it is preferably a *Campanula-*derived F3'5'H gene or its homolog, and selected from the group consisting of:
(a) a polynucleotide comprising the nucleotide sequence of SEQ ID NO: 7;
(b) a polynucleotide that hybridizes with a polynucleotide comprising the nucleotide sequence complementary to the nucleotide sequence of SEQ ID NO: 7 under stringent conditions and encodes a protein having the same activity as a protein encoded by the polynucleotide of (a);
(c) a polynucleotide encoding a protein comprising the amino acid sequence of SEQ ID NO: 8;
(d) a polynucleotide encoding a protein comprising an amino acid sequence that is the amino acid sequence of SEQ ID NO: 8 having a deletion, substitution, insertion and/or addition of one or more amino acids, and having the same activity as a protein encoded by the polynucleotide of (c); and
(e) a polynucleotide encoding a protein having an amino acid sequence with at least 90% identity with respect to the amino acid sequence of SEQ ID NO: 8 and having the same activity as a protein encoded by the polynucleotide of (c).

The source of the MT gene or its homolog is not particularly restricted so long as it has the desired function, but it is preferably a torenia-derived MT gene or its homolog, and selected from the group consisting of:
(a) a polynucleotide comprising the nucleotide sequence of SEQ ID NO: 9;
(b) a polynucleotide that hybridizes with a polynucleotide comprising the nucleotide sequence complementary to the nucleotide sequence of SEQ ID NO: 9 under stringent conditions and encodes a protein having the same activity as a protein encoded by the polynucleotide of (a);
(c) a polynucleotide encoding a protein comprising the amino acid sequence of SEQ ID NO: 10;
(d) a polynucleotide encoding a protein comprising an amino acid sequence that is the amino acid sequence of SEQ ID NO: 10 having a deletion, substitution, insertion and/or addition of one or more amino acids, and having the same activity as a protein encoded by the polynucleotide of (c); and
(e) a polynucleotide encoding a protein having an amino acid sequence with at least 90% identity with respect to the amino acid sequence of SEQ ID NO: 10 and having the same activity as a protein encoded by the polynucleotide of (c).

The term "polynucleotide" as used herein refers to DNA or RNA.

Also as used herein, the term "stringent conditions" refers to conditions that allow specific binding between a polynucleotide or oligonucleotide and genomic DNA in a selective and detectable manner. Stringent conditions are defined by an appropriate combination of salt concentration, organic solvent (for example, formamide), temperature and other known conditions. Specifically, stringency is increased by reducing the salt concentration, increasing the organic solvent concentration or raising the hybridization temperature. Stringency is also affected by the rinsing conditions after hybridization. The rinsing conditions are defined by the salt concentration and temperature, and stringency of rinsing is increased by reducing the salt concentration and raising the temperature. Therefore, the term "stringent conditions" means conditions such that specific hybridization takes place only between nucleotide sequences with high identity, such as a degree of "identity" between the nucleotide sequences of about 80% or greater, preferably about 90% or greater, more preferably about 95% or greater, even more preferably 97% or greater and most preferably 98% or greater, on average. The "stringent conditions" may be, for example, a temperature of 60°C to 68°C, a sodium concentration of 150 to 900 mM and preferably 600 to 900 mM, and a pH of 6 to 8, with specific examples including hybridization under conditions of 5 × SSC (750 mM NaCl, 75 mM trisodium citrate), 1% SDS, 5 × Denhardt solution, 50% formaldehyde, 42°C, and rinsing under conditions of 0.1 × SSC (15 mM NaCl, 1.5 mM trisodium citrate), 0.1% SDS, 55°C.

The hybridization may be carried out by a method that is publicly known in the field or a similar method, such as the method described in Current Protocols in Molecular Biology (edited by Frederick M. Ausubel et al., 1987). When a commercially available library is to be used, the hybridization may be carried out according to the method described in the accompanying directions for use. The gene selected by hybridization may be naturally derived, such as plant-derived or non-plant-derived. The gene selected by the hybridization may be cDNA, genomic DNA or chemically synthesized DNA.

The phrase "amino acid sequence having a deletion, substitution, insertion and/or addition of one or more amino acids" means an amino acid sequence having a deletion, substitution, insertion and/or addition of 1 to 20, preferably 1 to 5 and more preferably 1 to 3 arbitrary amino acids. Site-specific mutagenesis is a useful genetic engineering method as it allows introduction of specific mutations into specified sites, and it may be carried out by the method described in Molecular Cloning: A Laboratory Manual, 2nd Ed., Cold Spring Harbor Laboratory Press, Cold Spring Harbor, N.Y., 1989. By expressing the mutant DNA using a suitable expression system, it is possible to obtain a protein consisting of an amino acid sequence having a deletion, substitution, insertion and/or addition of one or more amino acids.

A polynucleotide can be obtained by a method that is publicly known to those skilled in the art, such as a method of chemical synthesis using the phosphoramidite method, or a nucleic acid amplification method using a plant nucleic acid specimen as template, and primers designed based on the nucleotide sequence of the target gene.

Throughout the present specification, the term "identity" means, for polypeptide sequences (or amino acid sequences) or polynucleotide sequences (or nucleotide sequences), the quantity (number) of amino acid residues or nucleotides composing them that can be determined to be identical between the two chains, in the sense of mutual agreement between them, to signify the degree of sequence correlation between two polypeptide sequences or two polynucleotide sequences, and this "identity" can be easily calculated. Numerous methods are known for measuring identity between two polynucleotide sequences or polypeptide sequences, and the term "identity" is well known to those skilled in the art (for example, see Lesk, A.M. (Ed.), Computational Molecular Biology, Oxford University Press, New York, (1988); Smith, D.W. (Ed.), Biocomputing: Informatics and Genome Projects, Academic Press, New York, (1993); Grifin, A.M. & Grifin, H.G. (Ed.), Computer Analysis of Sequence Data: Part I, Human Press, New Jersey, (1994); von Heinje, G., Sequence Analysis in Molecular Biology, Academic Press, New York, (1987); Gribskov, M. & Devereux, J. (Ed.), Sequence Analysis Primer, M-Stockton Press, New York, (1991) and elsewhere).

The numerical values for "identity" used in the present specification, unless otherwise specified, may be the numerical values calculated using an identity search program known to those skilled in the art, but they are preferably numerical values calculated using the ClustalW program of MacVector Application (version 9.5, Oxford Molecular Ltd., Oxford, England). According to the invention, the degree of "identity" between amino acid sequences is, for example, about 90% or greater, preferably about 95% or greater, more preferably about 97% or greater, and most preferably about 98% or greater.

The polynucleotide (nucleic acid, gene) of the invention "encodes" a protein of interest. Here, "encodes" means that it allows expression of the protein of interest in a state in which it exhibits its activity. The term "encodes" also includes both encoding a structural sequence (exon) that is a continuous section of the protein of interest, and encoding via an intervening sequence (intron).

A gene with a natural nucleotide sequence can be obtained by analysis using a DNA sequencer, for example. DNA encoding an enzyme having a modified amino acid sequence can also be synthesized using common site-specific mutagenesis or PCR, based on DNA having the natural nucleotide sequence. For example, a DNA fragment to be modified may be obtained by restriction enzyme treatment of natural cDNA or genomic DNA, and used as template for site-specific mutagenesis or PCR using primers with the desired mutation, to obtain a DNA fragment having the desired modification. The DNA fragment having the mutation may then be linked with a DNA fragment encoding another portion of the target enzyme.

Alternatively, in order to obtain DNA encoding an enzyme consisting of a shortened amino acid sequence, DNA encoding an amino acid sequence longer than the target amino acid sequence, such as the full-length amino acid sequence, may be cut with a desired restriction enzyme, and if the obtained DNA fragment does not code for the full target amino acid sequence, then a DNA fragment consisting of the sequence of the missing portion may be synthesized and linked to it.

By expressing the obtained polynucleotide using a gene expression system in *Escherichia coli* or yeast and measuring the enzyme activity, it is possible to confirm that the obtained polynucleotide encodes a protein with the desired activity.

The present invention relates to a (recombinant) vector, and especially an expression vector, including the aforementioned polynucleotide, and to chrysanthemum plants transformed by the vector.

The vector of the invention also comprises an expression control region, such as a promoter, terminator and replication origin, that are dependent on the type of host plant into which it is introduced. Examples of promoters that constitutively express polynucleotides in plant cells include cauliflower mosaic virus 35S promoter, El₂35S promoter having two 35S promoter enhancer regions linked together, and rd29A gene promoter, rbcS promoter and mac-1 promoter. For tissue-specific gene expression, a promoter for a gene expressed specifically in that tissue may be used.

The vector may be created by a common method using a restriction enzyme and ligase. Transformation of a host plant using the expression vector may also be carried out by a common method.

At the current level of technology it is possible to use techniques to introduce a polynucleotide into a plant and express the polynucleotide constitutively or in a tissue-specific manner. Transfer of the DNA into the plant may be carried out by a method known to those skilled in the art, such as the *Agrobacterium* method, binary vector method, electroporation method, PEG method or particle gun method.

Plants to be used as hosts for the invention are not particularly restricted and may be plants belonging to genus Rosaceae *Rosa,* Solanaceae *Petunia,* Compositae *Chrysanthemum,* Caryophyllaceae *Dianthus* (such as carnation) or Liliaceae *Lilium,* among which rose cultivar of Rosaceae *Rosa* (scientific name: *Rosa hybrida*) is especially preferred. The term "rose plant", as used herein, is a rose cultivar of Rosaceae *Rosa* (scientific name: *Rosa hybrida*), which is its taxonomical classification. Roses are largely classified as Hybrid Tea, Floribunda and Polyantha roses based on their tree form and flower size, with the major pigment (anthocyanin) in the petals of all lines being of two types, the cyanidin-type and pelargonidin-type. The type of rose plant used as a host for the invention is not particularly restricted, and any of these varieties or lines are suitable. Examples of rose varieties to be used as hosts include Ocean Song, Noblesse, Rita Perfumera, Cool Water, Fame, Topless and Peach Avalanche.

According to the invention it is possible to obtain a transgenic plant, preferably of Rosaceae *Rosa,* Solanaceae *Petunia,* Compositae *Chrysanthemum* or Caryophyllaceae *Dianthus,* (carnation), and most preferably a rose plant, having modified flower color, wherein a delphinidin-type anthocyanin and a flavone C-glycoside are copresent in the cells. Particularly when the obtained transgenic plant is a rose plant, it exhibits a flower color in the Blue group or Violet-Blue group according to the RHS Color Chart, and/or with a hue angle of 339.7° to 270.0° in the CIEL*a*b* color system.

The invention still further relates to cut flowers of the obtained transgenic plant or its inbred or outbred progeny, or the propagules, partial plant body, tissue or cells, or a processed form created from the cut flowers (especially processed cut flowers). The processed cut flowers referred to here include pressed flowers formed using cut flowers, or preserved flowers, dry flowers or resin sealed products, with no limitation to these.

The present invention will now be explained in greater detail by examples.

### EXAMPLES

### [Example 1: Simulation of flavone C-glycoside copigment effect with anthocyanin (malvin)]

An anthocyanin (malvin) and flavone C-glycoside were prepared to simulate the copigment effect of the flavone C-glycoside on malvin. The malvin (malvidin 3,5-diglucoside) and flavone C-glycosides (isovitexin (apigenin 6-C-glucoside), isoorientin (luteolin 6-C-glucoside) and swertisin (genkwanin 6-C-glucoside) used in the experiments were purchased from Nacalai Tesque, Inc. Embigenin (7,4'-dimethoxyisovitexin) was prepared by artificial organic synthesis from isovitexin. Each flavone C-glycoside (isovitexin, isoorientin, swertisin or embigenin) was added to the malvin at 10-equivalent molar concentration in a buffering solution at pH 5.0, and the absorption spectrum was measured. The malvin concentration was 0.5 mM.

Addition of the flavone C-glycoside increased the absorbance of the malvin solution, shifting the absorption maximum (λmax) toward the long wavelength end (toward blue). Shifting of the absorption maximum toward the long wavelength end was greatest with embigenin, demonstrating that embigenin exhibits the highest copigment effect with respect to malvin.

**[Table 1]**

| Absorption maximum of malvin solution upon addition of flavone C-glvcoside (λmax) | | |
|---|---|---|
| | | Malvin (malvidin 3,5-diglucoside) |
| Flavone *C*-glycoside (mg/g) | Embigenin | 581 nm |
| | Swertisin | 579 nm |
| | Isovitexin | 578 nm |
| | Isoorientin | 575 nm |
| Anthocyanin | Malvin | 529 nm |

### [Example 2: Detection of embigenin in fringed iris]

It has already been reported that embinin (embigenin 2"-rhamnoside) is present in fringed iris (*Iris japonica*) petals (https://www.jstage.jst.go.jp/article/yakushil947/93/12/93_12_1655/_pdf). Pigment analysis was carried out to confirm whether the embinin precursor embigenin is detected in fringed iris petals and leaves.

After freezing fringed iris petal and leaf samples, they were dried overnight with a vacuum freeze drier VirTis sentry 2.0 (SP Scientific), and then gently crushed with a spatula. To the crushed sample there was added 4 mL of 0.1% trifluoroacetic acid (TFA)-containing 50% acetonitrile per 10 mg of dry weight, and after treatment with ultrasonic waves for 20 minutes, the mixture was centrifuged (3,600 rpm, 4°C, 10 minutes) and the supernatant was recovered. The obtained supernatant was filtered with a 0.45 µm filter (COSMO NICE filter (aqueous), 0.45 µm, 13 mm). A 200 µL portion was dried, and after addition of β-glucosidase and naringinase it was treated overnight at 30°C, and then 200 µL of 0.1% TFA-containing 90% acetonitrile was added to suspend the reaction. It was then treated for 2 hours with ultrasonic waves, after which it was centrifuged (15,000 rpm, 4°C, 5 minutes) and the obtained supernatant was filtered with a 0.45 µm filter (Milex-LH, 0.45 µm, Millipore) and supplied to high-performance liquid chromatography. The analysis conditions were as follows.

### <Analysis conditions>

Apparatus: Prominence HPLC system (product of Shimadzu Corp.)
Detector: SPD-M20A (250 to 450 nm)
Column: Shim-pack FC-ODS 150 × 4.6 mm, 3 µm (Shimadzu GLC)
Eluent A: 0.1% TFA aqueous solution
Eluent B: 0.1 % TFA-containing 90% acetonitrile
Flow rate: 0.6 mL/min

Elution was performed using solution A (0.1% TFA aqueous solution) and solution B (aqueous 90% acetonitrile solution containing 0.1% TFA). The elution was with a 20-minute linear concentration gradient from a 9:1 mixture to a 8:2 mixture, a 15-minute linear concentration gradient from a 8:2 mixture to a 2:8 mixture and a 5-minute linear concentration gradient from a 2:8 mixture to a 0:10 mixture, followed by elution with a 0:10 mixture for 1 minute. The flow rate was 0.6 mL/min. Based on the analysis results obtained by the method described above, embigenin was detected only in the fringed iris petals (6.6 mg per 1 g dry weight).

**[Table 2]**

| Sample | Embigenin (mg content per 1 g dry weight) |
|---|---|
| Petals | 6.6 |
| Leaves | 0.0 |

### [Example 3: Acquisition of candidate genes coding for proteins having activity of transferring methyl group to 4'-position hydroxyl group of flavone C-glycoside]

### <Isolation of total RNA>

Using an RNeasy Plant Mini Kit (Qiagen Co.), total RNA was isolated from fringed iris petals and leaves by the manufacturer's recommended method.

### <Fringed iris cDNA expression analysis>

A library was prepared from the total RNA for provision to the next-generation sequencer NextSeq 500, using a SureSelect Strand-Specific RNA library preparation kit (Agilent Technologies) according to the manufacturer's recommended protocol. The prepared library was sequenced using a NextSeq 500 (Illumina Co.), and the obtained reads were analyzed. The reads from all of the samples were then combined and assembled using a Trinity v2.8.5, to determine the contig sequences. The obtained contig sequences were used for mapping of pair reads for each sample using an RSEM 1.3.0, and the FPKM value was calculated to determine the expression level.

### <Estimation of gene function>

The contig sequence was used for a BLAST search with NCBI NR and Araport11, followed by function annotation (gene function estimation).

### <Acquiring full-length cDNA of candidate gene>

From the obtained contig sequence there were obtained 612 candidate genes using "methyltransferase" as the search keyword, and then a BLAST search was conducted using the sequence of the oboushibana flavone 7-O-methyltransferase gene, acquiring an additional 25 candidate genes. The contig sequences were screened and narrowed down to 9 candidates having high expression and being expressed primarily in the petals. After constructing a phylogenetic tree with addition of 37 previously reported methyltransferase genes, including the barley flavonoid 7-O-methyltransferase gene (F1-OMT, NPL 10) and the burr medic isoflavone 7-O-methyltransferase gene (MtIOMT2, NPL 11), DN144 was selected as the candidate gene. Primers were designed based on the assembled full-length cDNA sequence, and full-length cDNA clones were obtained by the following method.

The cDNA was synthesized with the isolated fringed iris petal total RNA as template, using a SuperScript First-Strand Synthesis System for RT-PCR (ThermoFisher Scientific) according to the manufacturer's recommended protocol. With the obtained fringed iris petal cDNA as template, a PrimeSTAR Max (Takara Bio, Inc.) was used according to the manufacturer's recommend protocol for PCR reaction with a 50 µl reaction volume (30 cycles were repeated, with one cycle being: 98°C for 10 seconds, 55°C for 5 seconds and 72°C for 15 seconds, and were followed by holding at 4°C). The DN144 nucleotide sequence obtained in this manner was determined using a DNA sequencer (3500 Genetic Analyzer by Applied Biosystems).

Upon examining the homology between DN144 and a known methyltransferase gene on the amino acid level, it was found to have 33% homology with barley (*Hordeum vulgare*)*-*derived flavonoid 7-O-methyltransferase, 37% homology with burr medic (*Medicago tructula*)-derived isoflavone 7-O-methyltransferase (MtIOMT1), 36% homology with burr medic (*Medicago tructula*)-derived isoflavone/isoflavone 7-O-methyltransferase (MtIOMT2), 37% homology with gromwell burr medic (*Medicago sativa*)-derived isoflavone O-methyltransferase, and 36% homology with licorice (*Glycyrrhiza echinata*)-derived daidzein 7-O-methyltransferase. DN144 is therefore clearly distinguishable from known methyltransferase genes.

### [Example 4: Measurement of enzyme activity in E. coli by protein having activity of transferring methyl group to 4'-position hydroxyl group of flavone C-glycoside]

### <Construction of E. coli expression vector>

With DN144 as a candidate protein having activity of transferring a methyl group to the 4'-position hydroxyl group of flavone C-glycoside, and using pET15b (Novagen), an *E. coli* expression vector pSPB7942 containing the full-length of DN144 was constructed according to the manufacturer's recommended protocol.

### <Expression of methyltransferase in E. coli>

Vector pSPB7942 was introduced into *E. coli* BL21(DE3) (New England Biolabs Japan Inc) by the manufacturer's recommended protocol, to obtain *E. coli* transformants. The *E. coli* were cultured using an Overnight Express Autoinduction System 1 (Novagen), according to the manufacturer's recommended protocol. The *E. coli* transformants were cultured at 37°C (approximately 4 hours) with 2 mL of prepared culture solution, to an OD600 value of 0.5. The *E. coli* solution was added as a preculturing solution to 50 mL of culture solution, and main culturing was carried out for two nights at 16°C. The *E. coli* solution cultured for two nights was centrifuged (3,000 rpm, 4°C, 15 minutes), and the collected cells were suspended in sonic buffer (composition: 40 mM KPB (pH7.5), 1 mM dithiothreitol, 50 µM amidinophenylmethanesulfonyl fluoride hydrochloride, 500 µM ethylenediaminetetraacetic acid, 2 mM MgCl₂, 1 µM S-adenosylmethionine (SAM)). A 5 mL portion of sonic buffer was added for each 1 g of *E. coli.* The suspended *E. coli* cells were crushed by ultrasonic treatment and then centrifuged (15,000 rpm, 4°C, 10 minutes), and the supernatant was recovered. The supernatant was used as a crude protein solution extract from *E. coli* expressing DN144. A TOMY MX-307 (rotor: AR015-24) was used for centrifugal separation (Tomy Seiko Co., Ltd.).

### <Enzyme activity measurement>

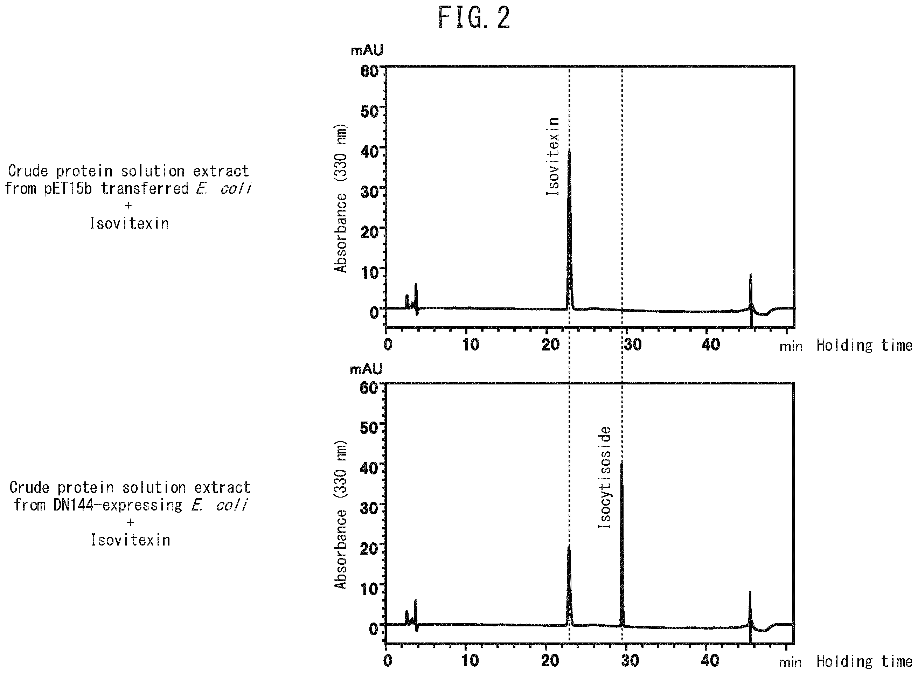

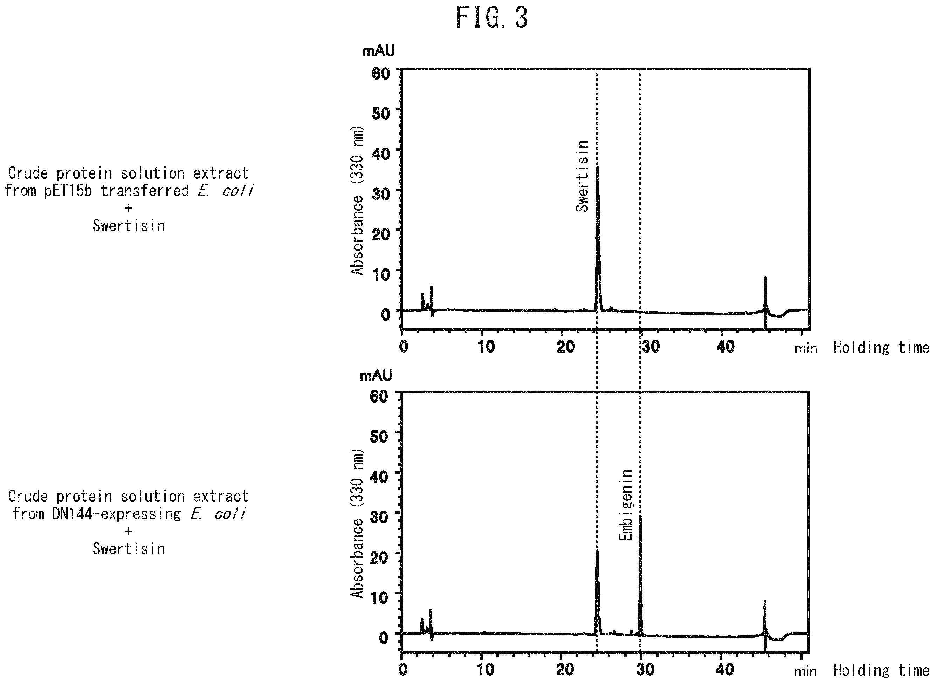

The substrates used for activity measurement were apigenin, apigenin 7-glucoside, isovitexin, isovitexin 2"-rhamnoside, saponarin (isovitexin 4'-glucoside), swertisin, luteolin, luteolin 7-glucoside, isoorientin, swertiajaponin (isoorientin 7-methyl ether), delphinidin 3-glucoside, delphinidin 3,5-diglucoside and malvidin 3,5-diglucoside. A mixture of 8 µL of each 1 mM substrate (50% acetonitrile aqueous
solution containing 0.1% TFA), 20 µL of 10 mM SAM, 10 µL of 1 M KPB (pH 7.5) and 20 µL of 10 mM MgCl₂ adjusted to 58 µL was kept at 30°C for 10 minutes, and then 42 µL of crude protein solution extract from DN144-expressing *E. coli* was added for enzyme reaction (30 minutes at 30°C). Next, 100 µL of stop buffer (aqueous 90% acetonitrile solution containing 0.1% TFA) was added to stop the enzyme reaction, and the enzyme reaction solution was analyzed by high-performance liquid chromatography (LC-2030C by Shimadzu Corp.). Detection was at 330 nm using a Shimadzu PDA SPD-M20A as the detector. The column used as a Shim-Pack FC-ODS 150 mm∗4.6 mm (Shimadzu GLC). Elution was performed using solution A (0.1% TFA aqueous solution) and solution B (aqueous 90% acetonitrile solution containing 0.1% TFA). The elution was with a 20-minute linear concentration gradient from a 9:1 mixture to a 8:2 mixture, a 15-minute linear concentration gradient from a 8:2 mixture to a 2:8 mixture and a 5-minute linear concentration gradient from a 2:8 mixture to a 0:10 mixture, followed by elution with a 0:10 mixture for 1 minute. The flow rate was 0.6 mL/min. As a control, the same experiment was carried out using a crude protein solution extract from *E. coli* in which pET15b vector without the insert had been introduced. As a result, compounds were detected in the enzyme reaction solutions obtained by reacting the 10 different flavones with the crude protein solution extract from the DN144-expressing *E. coli.* Particularly high activity was exhibited for isovitexin 2"-rhamnoside, isoorientin and swertia japonin, with 75 to 100% being converted to the compounds. Using samples, conversion to isocytisoside was confirmed with isovitexin as substrate, and conversion to embigenin was confirmed with swertisin as substrate (see Table 3, Figs. 2 and 3). With reaction of the 3 different anthocyanins, no peaks were detected other than for the substrates in the enzyme reaction solution. When enzyme reaction was conducted using an anthocyanin substrate, the enzyme reaction conditions were 30°C, 15 minutes, and an aqueous 90% acetonitrile solution containing 0.1% TFA and 0.24 N hydrochloric acid was used as the stop buffer. A Shimadzu PDA SPD-M20A detector was used for detection at 520 nm during analysis of the enzyme reaction solution by high-performance liquid chromatography (Prominence (Shimadzu Corp.)). The column used was a Shodex RSpak DE-413L (Showa Denko K.K.). Elution was performed using solution A (0.5% TFA aqueous solution) and solution B (aqueous 50% acetonitrile solution containing 0.5% TFA). Elution was performed with a 15-minute linear concentration gradient from an 8:2 mixture to a 0:10 mixture, and subsequent elution for 5 minutes with a 0:10 mixture. The flow rate was 0.6 mL/min.

**[Table 3]**

| Flavonoid type | Type | Substrate | Produced compound |
|---|---|---|---|
| Flavone | Apigenin-type | Apigenin | Compound produced (unidentified) |
| | | Apigenin 7-glucoside | Compound produced (unidentified) |
| | | Isovitexin | Isocytisoside |
| | | Isovitexin 2"-rhamnoside | Compound produced (unidentified) |
| | | Saponarin | Compound produced (unidentified) |
| | | Swertisin | Embigenin |
| | Luteolin-type | Luteolin | Compound produced (unidentified) |
| | | Luteolin 7-glucoside | Compound produced (unidentified) |
| | | Isoorientin | Compound produced (unidentified) |
| | | Swertiaj aponin | Compound produced (unidentified) |
| Anthocyanin | | Delphinidin 3-glucoside | N.D. |
| | Delphinidin-type | Delphinidin 3,5-diglucoside | N.D. |
| | Malvidin-type | Malvidin 3,5-diglucoside | N.D. |

| | | | |
|---|---|---|---|
| N.D. : Not detected | | | |

The results clearly showed that DN144 exhibits activity of specifically transferring a methyl group to the 4'-position hydroxyl group of flavone C-glycoside, indicating that DN144 is a gene coding for a protein having activity of transferring a methyl group to the 4'-position hydroxyl group of flavone C-glycoside. DN144 also exhibited strong methyltransferase activity for isovitexin 2"-rhamnoside, isoorientin and swertiajaponin.

Based on these results, the gene was identified as a gene coding for a protein having activity of transferring a methyl group to the 4'-position hydroxyl group of flavone C-glycoside, and was designated as IjFn-4'OMT.

### [Example 5: (Route 1) Transfer of Campanula-derived F3'5'H gene, torenia-derived MT gene, licorice-derived F2H gene, buckwheat-derived codon usage-modified flavone CGT gene, Lotus japonicus-derived FDH gene, oboushibana-derived F7OMT gene and fringed iris-derived F4'OMT gene into rose variety "Ocean Song"]

Plasmid pSPB7964 has pBINPLUS as the basic backbone, and contains the following seven expression cassettes.
(1) El₂35S promoter, *Campanula-*derived F3'5'H full-length cDNA (SEQ ID NO: 7) and D8 terminator
(2) El₂35S promoter, torenia-derived MT full-length cDNA (SEQ ID NO: 9) and *Arabidopsis thaliana*-derived HSP terminator
(3) El₂35S promoter, licorice-derived F2H full-length cDNA (SEQ ID NO: 12) and perilla-derived AT terminator
(4) El₂35S promoter, buckwheat-derived codon usage-modified flavone CGT full-length cDNA (SEQ ID NO: 16) (*Arabidopsis thaliana* HSPRO gene-derived 5'-UTR (SEQ ID NO: 18) attached to the 5'-position end) and *Arabidopsis thaliana-*derived HSP terminator
(5) 35S promoter, *Lotus japonicus*-derived FDH full-length cDNA (SEQ ID NO: 14) and *Arabidopsis thaliana-*derived HSP terminator
(6) El₂35S promoter, oboushibana-derived F7OMT full-length cDNA (SEQ ID NO: 1) and *Arabidopsis thaliana-*derived HSP terminator
(7) El₂35S promoter, fringed iris-derived F4'OMT full-length cDNA (SEQ ID NO: 19) and *Arabidopsis thaliana-*derived HSP terminator

This plasmid constitutively expresses the *Campanula* F3'5'H gene, torenia MT gene, licorice F2H gene, buckwheat codon usage-modified flavone CGT gene, *Lotus japonicus* FDH gene, oboushibana F7OMT gene and fringed iris F4'OMT gene in plants.

The constructed plasmid pSPB7964 was introduced into the blue rose variety "Ocean Song".

### [Example 6: (Route 1) Transfer of Campanula-derived F3'5'H gene, torenia-derived MT gene, licorice-derived F2H gene, buckwheat-derived codon usage-modified flavone CGT gene, Lotus japonicus-derived FDH gene, oboushibana-derived F7OMT gene and fringed iris-derived codon usage-modified F4'OMT gene into rose variety "Ocean Song"]

Plasmid pSPB7965 has pBINPLUS as the basic backbone, and contains the following seven expression cassettes.
(1) El₂35S promoter, *Campanula-*derived F3'5'H full-length cDNA (SEQ ID NO: 7) and D8 terminator
(2) El₂35S promoter, torenia-derived MT full-length cDNA (SEQ ID NO: 9) and *Arabidopsis thaliana-*derived HSP terminator
(3) El₂35S promoter, licorice-derived F2H full-length cDNA (SEQ ID NO: 12) and perilla-derived AT terminator
(4) El₂35S promoter, buckwheat-derived codon usage-modified flavone CGT full-length cDNA (SEQ ID NO: 16) (*Arabidopsis thaliana* HSPRO gene-derived 5'-UTR (SEQ ID NO: 18) attached to the 5'-position end) and *Arabidopsis thaliana-*derived HSP terminator
(5) 35S promoter, *Lotus japonicus-*derived FDH full-length cDNA (SEQ ID NO: 14) and *Arabidopsis thaliana-*derived HSP terminator
(6) El₂35S promoter, oboushibana-derived F7OMT full-length cDNA (SEQ ID NO: 1) and *Arabidopsis thaliana-*derived HSP terminator
(7) El₂35S promoter, fringed iris-derived codon usage-modified F4'OMT full-length cDNA (SEQ ID NO: 21) and *Arabidopsis thaliana-*derived HSP terminator

This plasmid constitutively expresses the *Campanula* F3'5'H gene, torenia MT gene, licorice F2H gene, buckwheat codon usage-modified flavone CGT gene, *Lotus japonicus* FDH gene, oboushibana F7OMT gene and fringed iris codon usage-modified F4'OMT gene in plants.

The constructed plasmid pSPB7965 was introduced into the blue rose variety "Ocean Song".

### [Example 7: (Route 2) Transfer of Campanula-derived F3'5'H gene, torenia-derived MT gene, torenia-derived FNS gene, gentian-derived flavone CGT gene, oboushibana-derived F7OMT gene and fringed iris-derived F4'OMT gene into rose variety "Ocean Song"]

Plasmid pSPB7960 has pBINPLUS as the basic backbone, and contains the following six expression cassettes.
(1) El₂35S promoter, *Campanula-*derived F3'5'H full-length cDNA (SEQ ID NO: 7) and D8 terminator
(2) El₂35S promoter, torenia-derived MT full-length cDNA (SEQ ID NO: 9) and *Arabidopsis thaliana-*derived HSP terminator
(3) El₂35S promoter, torenia-derived FNS full-length cDNA (SEQ ID NO: 3) and D8 terminator
(4) El₂35S promoter, gentian-derived flavone CGT full-length cDNA (SEQ ID NO: 5) (*Arabidopsis thaliana* alcohol dehydrogenase (ADH) gene-derived 5'-UTR (SEQ ID NO: 11) attached to the 5'-position end) and *Arabidopsis thaliana-*derived HSP terminator
(5) El₂35S promoter, oboushibana-derived F7OMT full-length cDNA (SEQ ID NO: 1) and *Arabidopsis thaliana-*derived HSP terminator
(6) El₂35S promoter, fringed iris-derived F4'OMT full-length cDNA (SEQ ID NO: 19) and *Arabidopsis thaliana-*derived HSP terminator

This plasmid constitutively expresses the *Campanula* F3'5'H gene, torenia MT gene, torenia FNS gene, gentian flavone CGT gene, oboushibana F7OMT gene and fringed iris F4'OMT gene in plants.

The constructed plasmid pSPB7960 was introduced into the blue rose variety "Ocean Song".

### [Example 8: (Route 2) Transfer of Campanula-derived F3'5'H gene, torenia-derived MT gene, torenia-derived FNS gene, gentian-derived flavone CGT gene, oboushibana-derived F7OMT gene and fringed iris-derived codon usage-modified F4'OMT gene into rose variety "Ocean Song"]

Plasmid pSPB7961 has pBINPLUS as the basic backbone, and contains the following six expression cassettes.
(1) El₂35S promoter, *Campanula-*derived F3'5'H full-length cDNA (SEQ ID NO: 7) and D8 terminator
(2) El₂35S promoter, torenia-derived MT full-length cDNA (SEQ ID NO: 9) and *Arabidopsis thaliana-*derived HSP terminator
(3) El₂35S promoter, torenia-derived FNS full-length cDNA (SEQ ID NO: 3) and D8 terminator
(4) El₂35S promoter, gentian-derived flavone CGT full-length cDNA (SEQ ID NO: 5) (*Arabidopsis thaliana* alcohol dehydrogenase (ADH) gene-derived 5'-UTR (SEQ ID NO: 11) attached to the 5'-position end) and *Arabidopsis thaliana-*derived HSP terminator
(5) El₂35S promoter, oboushibana-derived F7OMT full-length cDNA (SEQ ID NO: 1) and *Arabidopsis thaliana-*derived HSP terminator
(6) El₂35S promoter, fringed iris-derived codon usage-modified F4'OMT full-length cDNA (SEQ ID NO: 21) and *Arabidopsis thaliana-*derived HSP terminator

This plasmid constitutively expresses the *Campanula* F3'5'H gene, torenia MT gene, torenia FNS gene, gentian flavone CGT gene, oboushibana F7OMT gene and fringed iris codon usage-modified F4'OMT gene in plants.

The constructed plasmid pSPB7961 was introduced into the blue rose variety "Ocean Song".

### [Example 9: Expression in petunia of gene coding for protein having activity of transferring methyl group to 4'-position hydroxyl group of flavone (1)]

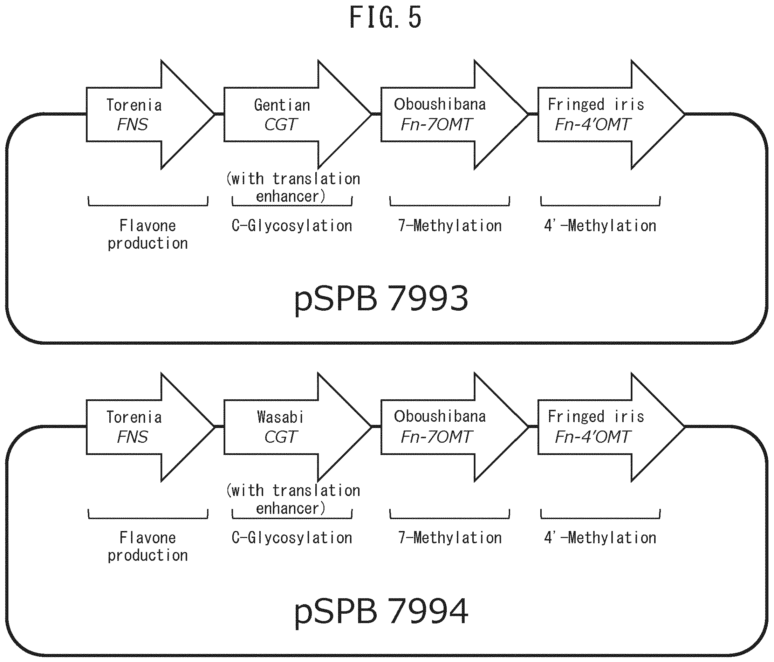

The binary vector pSPB7993 containing the IjFn-4'OMT gene of the invention was constructed to confirm whether or not the IjFn-4'OMT gene has activity of transferring a methyl group to the 4'-position hydroxyl group of flavones in plants (Fig. 5, top). The vector had pBINPLUS as the basic backbone, and contained the following four expression cassettes.
(1) El₂35S promoter, torenia-derived FNS full-length cDNA (SEQ ID NO: 3) and D8 terminator
(2) El₂35S promoter, gentian-derived CGT full-length cDNA (SEQ ID NO: 5) (*Arabidopsis thaliana* ADH gene-derived 5'-UTR (SEQ ID NO: 11) attached) and *Arabidopsis thaliana-*derived HSP terminator
(3) El₂35S promoter, oboushibana-derived CcFn-7OMT full-length cDNA (SEQ ID NO: 1) and *Arabidopsis thaliana-*derived HSP terminator
(4) El₂35S promoter, fringed iris-derived IjFn-4'OMT full-length cDNA (SEQ ID NO: 19) and *Arabidopsis thaliana-*derived HSP terminator

This binary vector constitutively expresses the torenia FNS gene, gentian CGT gene, oboushibana CcFn-7OMT gene and fringed iris IjFn-4'OMT gene in plants.

Plasmid pSPB7993 constructed in this manner was introduced into the petunia variety "Surfinia Bouquet Red", and a total of seven transformants were obtained. As a result of pigment analysis, accumulation of embigenin (apigenin7,4'-dimethyl-6-C-glucoside) with methylation of the 7,4'-positions of flavone C-glycoside was confirmed in one of the transformants, the content of the 7,4'-dimethylated form with respect to the total flavone C-glycoside being 8.4% (Table 4).

**[Table 4]**

| Plant No. | 7,4'-Dimethylated (%) | Flavonol (mg/g) | | Flavone (mg/g) | | Flavone C-glycoside (mg/g) | | | | |
|---|---|---|---|---|---|---|---|---|---|---|
| | | Q | K | Lut | Api | IVX | Iori | Swe | Emb | Swaj |
| Host | 0.0 | 0.714 | 0.100 | 0.000 | 0.000 | 0.000 | 0.000 | 0.000 | 0.000 | 0.000 |
| 1 | 8.4 | 1.359 | 0.381 | 0.000 | 0.017 | 0.000 | 0.000 | 0.253 | 0.023 | 0.000 |

| | | | | | | | | | | |
|---|---|---|---|---|---|---|---|---|---|---|
| Host: Surfinia Bouquet Red Q: Quercetin, K: Kaempferol Lut: Luteolin, Api: Apigenin, IVX: Isovitexin, Iori: Isoorientin, Swe: Swertisin, Emb: Embigenin, Swaj: Swertiajaponin | | | | | | | | | | |

Swertisin (apigenin 7-methyl-6-C-glucoside) was detected in the line as a flavone C-glycoside in addition to the 7,4'-dimethylated forms. None of the flavone C-glycosides were detected in the host. This clearly showed that IjFn-4'OMT has activity of transferring a methyl group to the 4'-position of flavones in plants. By utilizing this gene it is possible to produce 4'-methylated forms of flavone C-glycosides in plants.

### [Example 10: Expression in petunia of gene coding for protein having activity of transferring methyl group to 4'-position hydroxyl group of flavone (2)]

The binary vector pSPB7994 containing the IjFn-4'OMT gene of the invention was constructed to confirm whether or not the IjFn-4'OMT gene has activity of transferring a methyl group to the 4'-position hydroxyl group of flavones in plants (Fig. 5, bottom). The vector had pBINPLUS as the basic backbone, and contained the following four expression cassettes.
(1) El₂35S promoter, torenia-derived FNS full-length cDNA (SEQ ID NO: 3) and D8 terminator
(2) El₂35S promoter, wasabi-derived CGT full-length cDNA (SEQ ID NO: 22) (*Arabidopsis thaliana* ADH gene-derived 5'-UTR (SEQ ID NO: 11) attached) and *Arabidopsis thaliana-*derived HSP terminator
(3) El₂35S promoter, oboushibana-derived CcFn-7OMT full-length cDNA (SEQ ID NO: 1) and *Arabidopsis thaliana-*derived HSP terminator
(4) El₂35S promoter, fringed iris-derived IjFn-4'OMT full-length cDNA (SEQ ID NO: 19) and *Arabidopsis thaliana-*derived HSP terminator

This binary vector constitutively expresses the torenia FNS gene, wasabi CGT gene, oboushibana CcFn-7OMT gene and fringed iris IjFn-4'OMT gene in plants.

Plasmid pSPB7994 constructed in this manner was introduced into the petunia variety "Surfinia Bouquet Red", and a total of 8 transformants were obtained. As a result of pigment analysis, accumulation of embigenin (apigenin 7,4'-dimethyl-6-C-glucoside) with methylation of the 7,4'-positions of flavone C-glycoside was confirmed in four of the transformants, the maximum content of the 7,4'-dimethylated form with respect to the total flavone C-glycoside being 9.5% (average content: 8.0%) (Table 5).

**[Table 5]**

| Plant No. | 7,4'-Dimethylated (%) | Flavonol (mg/g) | | Flavone (mg/g) | | Flavone C-glycoside (mg/g) | | | | |
|---|---|---|---|---|---|---|---|---|---|---|
| | | Q | K | Lut | Api | IVX | Iori | Swe | Emb | Swaj |
| Host | 0.0 | 0.714 | 0.100 | 0.000 | 0.000 | 0.000 | 0.000 | 0.000 | 0.000 | 0.000 |
| 1 | 7.1 | 0.472 | 0.048 | 0.000 | 0.000 | 0.000 | 0.000 | 0.849 | 0.071 | 0.082 |
| 2 | 9.5 | 0.496 | 0.108 | 0.000 | 0.000 | 0.000 | 0.000 | 0.558 | 0.059 | 0.000 |
| 3 | 6.7 | 1.251 | 0.155 | 0.000 | 0.000 | 0.000 | 0.000 | 0.540 | 0.044 | 0.076 |
| 4 | 8.8 | 0.791 | 0.063 | 0.000 | 0.000 | 0.000 | 0.000 | 0.796 | 0.085 | 0.086 |

| | | | | | | | | | | |
|---|---|---|---|---|---|---|---|---|---|---|
| Host: Surfinia Bouquet Red Q: Quercetin, K: Kaempferol Lut: Luteolin, Api: Apigenin, IVX: Isovitexin, Iori: Isoorientin, Swe: Swertisin, Emb: Embigenin, Swaj: Swertiajaponin | | | | | | | | | | |

Two different flavone C-glycosides, swertisin (apigenin 7-methyl-6-C-glucoside) and swertiajaponin (luteolin 7-methyl-6-C-glucoside), were also detected in the line in addition to the 7,4'-dimethylated forms. None of the flavone C-glycosides were detected in the host. This clearly showed that IjFn-4'OMT has activity of transferring a methyl group to the 4'-position of flavones in plants. By utilizing this gene it is possible to produce 4'-methylated forms of flavone C-glycosides in plants.

[Sequence Listing]

## Claims

1. A polynucleotide selected from the group consisting of the following (A) to (E):
(A) a polynucleotide comprising the nucleotide sequence of SEQ ID NO: 19 or SEQ ID NO: 21;
(B) a polynucleotide that hybridizes with a polynucleotide comprising the nucleotide sequence complementary to the nucleotide sequence of SEQ ID NO: 19 or SEQ ID NO: 21 under stringent conditions, and that encodes a protein having activity of transferring a methyl group to the 4'-position hydroxyl group of flavone C-glycoside;
(C) a polynucleotide encoding a protein comprising the amino acid sequence of SEQ ID NO: 20;
(D) a polynucleotide encoding a protein comprising an amino acid sequence that is the amino acid sequence of SEQ ID NO: 20 having a deletion, substitution, insertion and/or addition of one or more amino acids, and having activity of transferring a methyl group to the 4'-position hydroxyl group of flavone C-glycoside; and
(E) a polynucleotide encoding a protein comprising an amino acid sequence with at least 90% identity with respect to the amino acid sequence of SEQ ID NO: 20 and having activity of transferring a methyl group to the 4'-position hydroxyl group of flavone C-glycoside.

2. The polynucleotide according to claim 1, which is a polynucleotide comprising the nucleotide sequence of SEQ ID NO: 19 or SEQ ID NO: 21.

3. The polynucleotide according to claim 1, which is a polynucleotide encoding a protein comprising the amino acid sequence of SEQ ID NO: 20.

4. A protein encoded by the polynucleotide according to any one of claims 1 to 3.

5. A vector comprising a polynucleotide according to any one of claims 1 to 3.

6. A vector according to claim 5, further comprising a flavone 7-O-methyltransferase (Fn-7OMT) gene or its homolog.

7. A vector according to claim 6, wherein the Fn-7OMT gene or its homolog is selected from the group consisting of:
(a) a polynucleotide comprising the nucleotide sequence of SEQ ID NO: 1;
(b) a polynucleotide that hybridizes with a polynucleotide comprising the nucleotide sequence complementary to the nucleotide sequence of SEQ ID NO: 1 under stringent conditions, and that encodes a protein having activity of transferring a methyl group to the 7-position hydroxyl group of flavone C-glycoside;
(c) a polynucleotide encoding a protein comprising the amino acid sequence of SEQ ID NO: 2;
(d) a polynucleotide encoding a protein comprising an amino acid sequence that is the amino acid sequence of SEQ ID NO: 2 having a deletion, substitution, insertion and/or addition of one or more amino acids, and having activity of transferring a methyl group to the 7-position hydroxyl group of flavone C-glycoside; and
(e) a polynucleotide encoding a protein comprising an amino acid sequence with at least 90% identity with respect to the amino acid sequence of SEQ ID NO: 2 and having activity of transferring a methyl group to the 7-position hydroxyl group of flavone C-glycoside.

8. A vector according to any one of claims 5 to 7, further comprising a flavone synthase (FNS) gene or its homolog, and a flavone C-glycosyltransferase (CGT) gene or its homolog.

9. A vector according to claim 8, wherein
the FNS gene or its homolog is selected from the group consisting of:
(1-a) a polynucleotide comprising the nucleotide sequence of SEQ ID NO: 3;
(1-b) a polynucleotide that hybridizes with a polynucleotide comprising the nucleotide sequence complementary to the nucleotide sequence of SEQ ID NO: 3 under stringent conditions and encodes a protein having the same activity as a protein encoded by the polynucleotide of (1-a);
(1-c) a polynucleotide encoding a protein comprising the amino acid sequence of SEQ ID NO: 4;
(1-d) a polynucleotide encoding a protein comprising an amino acid sequence that is the amino acid sequence of SEQ ID NO: 4 having a deletion, substitution, insertion and/or addition of one or more amino acids, and having the same activity as a protein encoded by the polynucleotide of (1-c); and
(1-e) a polynucleotide encoding a protein having an amino acid sequence with at least 90% identity with respect to the amino acid sequence of SEQ ID NO: 4 and having the same activity as a protein encoded by the polynucleotide of (1-c), and
the flavone CGT gene or its homolog is selected from the group consisting of:
(2-a) a polynucleotide comprising the nucleotide sequence of SEQ ID NO: 5 or SEQ ID NO: 22;
(2-b) a polynucleotide that hybridizes with a polynucleotide comprising the nucleotide sequence complementary to the nucleotide sequence of SEQ ID NO: 5 or SEQ ID NO: 22 under stringent conditions and encodes a protein having the same activity as a protein encoded by the polynucleotide of (2-a);
(2-c) a polynucleotide encoding a protein comprising the amino acid sequence of SEQ ID NO: 6 or SEQ ID NO: 23;
(2-d) a polynucleotide encoding a protein comprising an amino acid sequence that is the amino acid sequence of SEQ ID NO: 6 or SEQ ID NO: 23 having a deletion, substitution, insertion and/or addition of one or more amino acids, and having the same activity as a protein encoded by the polynucleotide of (2-c); and
(2-e) a polynucleotide encoding a protein having an amino acid sequence with at least 90% identity with respect to the amino acid sequence of SEQ ID NO: 6 or SEQ ID NO: 23 and having the same activity as a protein encoded by the polynucleotide of (2-c).

10. A vector according to claim 8 or 9, further comprising a flavonoid F3'5' hydroxylase (F3'5'H) gene or its homolog, and a methyltransferase (MT) gene or its homolog.

11. The vector according to claim 10, wherein
the F3'5'H gene or its homolog is selected from the group consisting of:
(3-a) a polynucleotide comprising the nucleotide sequence of SEQ ID NO: 7;
(3-b) a polynucleotide that hybridizes with a polynucleotide comprising the nucleotide sequence complementary to the nucleotide sequence of SEQ ID NO: 7 under stringent conditions and encodes a protein having the same activity as a protein encoded by the polynucleotide of (3-a);
(3-c) a polynucleotide encoding a protein comprising the amino acid sequence of SEQ ID NO: 8;
(3-d) a polynucleotide encoding a protein comprising an amino acid sequence that is the amino acid sequence of SEQ ID NO: 8 having a deletion, substitution, insertion and/or addition of one or more amino acids, and having the same activity as a protein encoded by the polynucleotide of (3-c); and
(3-e) a polynucleotide encoding a protein having an amino acid sequence with at least 90% identity with respect to the amino acid sequence of SEQ ID NO: 8 and having the same activity as a protein encoded by the polynucleotide of (3-c), and
the MT gene or its homolog is selected from the group consisting of:
(4-a) a polynucleotide comprising the nucleotide sequence of SEQ ID NO: 9;
(4-b) a polynucleotide that hybridizes with a polynucleotide comprising the nucleotide sequence complementary to the nucleotide sequence of SEQ ID NO: 9 under stringent conditions and encodes a protein having the same activity as a protein encoded by the polynucleotide of (4-a);
(4-c) a polynucleotide encoding a protein comprising the amino acid sequence of SEQ ID NO: 10;
(4-d) a polynucleotide encoding a protein comprising an amino acid sequence that is the amino acid sequence of SEQ ID NO: 10 having a deletion, substitution, insertion and/or addition of one or more amino acids, and having the same activity as a protein encoded by the polynucleotide of (4-c); and
(4-e) a polynucleotide encoding a protein having an amino acid sequence with at least 90% identity with respect to the amino acid sequence of SEQ ID NO: 10 and having the same activity as a protein encoded by the polynucleotide of (4-c).

12. A vector according to any one of claims 9 to 11, wherein *Arabidopsis thaliana* alcohol dehydrogenase (ADH) gene 5'-untranslated region (5'-UTR) (SEQ ID NO: 11) is attached to the flavone CGT gene or its homolog.

13. A vector according to any one of claims 5 to 7, further comprising the flavanone 2-hydroxylase (F2H) gene or its homolog, the flavone C-glycosyltransferase (CGT) gene or its homolog, and the dehydratase (FDH) gene or its homolog.

14. A vector according to claim 13, further comprising a flavonoid F3'5' hydroxylase (F3'5'H) gene or its homolog, and the methyltransferase (MT) gene or its homolog.

15. A vector according to claim 14, wherein
the F2H gene or its homolog is selected from the group consisting of:
(5-a) a polynucleotide comprising the nucleotide sequence of SEQ ID NO: 12;
(5-b) a polynucleotide that hybridizes with a polynucleotide comprising the nucleotide sequence complementary to the nucleotide sequence of SEQ ID NO: 12 under stringent conditions and encodes a protein having the same activity as a protein encoded by the polynucleotide of (5-a);
(5-c) a polynucleotide encoding a protein comprising the amino acid sequence of SEQ ID NO: 13;
(5-d) a polynucleotide encoding a protein comprising an amino acid sequence that is the amino acid sequence of SEQ ID NO: 13 having a deletion, substitution, insertion and/or addition of one or more amino acids, and having the same activity as a protein encoded by the polynucleotide of (5-c); and
(5-e) a polynucleotide encoding a protein having an amino acid sequence with at least 90% identity with respect to the amino acid sequence of SEQ ID NO: 13 and having the same activity as a protein encoded by the polynucleotide of (5-c),
the flavone CGT gene or its homolog is selected from the group consisting of:
(6-a) a polynucleotide comprising the nucleotide sequence of SEQ ID NO: 16;
(6-b) a polynucleotide that hybridizes with a polynucleotide comprising the nucleotide sequence complementary to the nucleotide sequence of SEQ ID NO: 16 under stringent conditions and encodes a protein having the same activity as a protein encoded by the polynucleotide of (6-a);
(6-c) a polynucleotide encoding a protein comprising the amino acid sequence of SEQ ID NO: 17;
(6-d) a polynucleotide encoding a protein comprising an amino acid sequence that is the amino acid sequence of SEQ ID NO: 17 having a deletion, substitution, insertion and/or addition of one or more amino acids, and having the same activity as a protein encoded by the polynucleotide of (6-c); and
(6-e) a polynucleotide encoding a protein having an amino acid sequence with at least 90% identity with respect to the amino acid sequence of SEQ ID NO: 17 and having the same activity as a protein encoded by the polynucleotide of (6-c),
the FDH gene or its homolog is selected from the group consisting of:
(7-a) a polynucleotide comprising the nucleotide sequence of SEQ ID NO: 14;
(7-b) a polynucleotide that hybridizes with a polynucleotide comprising the nucleotide sequence complementary to the nucleotide sequence of SEQ ID NO: 14 under stringent conditions and encodes a protein having the same activity as a protein encoded by the polynucleotide of (7-a);
(7-c) a polynucleotide encoding a protein comprising the amino acid sequence of SEQ ID NO: 15;
(7-d) a polynucleotide encoding a protein comprising an amino acid sequence that is the amino acid sequence of SEQ ID NO: 15 having a deletion, substitution, insertion and/or addition of one or more amino acids, and having the same activity as a protein encoded by the polynucleotide of (7-c); and
(7-e) a polynucleotide encoding a protein having an amino acid sequence with at least 90% identity with respect to the amino acid sequence of SEQ ID NO: 15 and having the same activity as a protein encoded by the polynucleotide of (7-c),
the F3'5'H gene or its homolog is selected from the group consisting of:
(8-a) a polynucleotide comprising the nucleotide sequence of SEQ ID NO: 7;
(8-b) a polynucleotide that hybridizes with a polynucleotide comprising the nucleotide sequence complementary to the nucleotide sequence of SEQ ID NO: 7 under stringent conditions and encodes a protein having the same activity as a protein encoded by the polynucleotide of (8-a);
(8-c) a polynucleotide encoding a protein comprising the amino acid sequence of SEQ ID NO: 8;
(8-d) a polynucleotide encoding a protein comprising an amino acid sequence that is the amino acid sequence of SEQ ID NO: 8 having a deletion, substitution, insertion and/or addition of one or more amino acids, and having the same activity as a protein encoded by the polynucleotide of (8-c); and
(8-e) a polynucleotide encoding a protein having an amino acid sequence with at least 90% identity with respect to the amino acid sequence of SEQ ID NO: 8 and having the same activity as a protein encoded by the polynucleotide of (8-c), and
the MT gene or its homolog is selected from the group consisting of:
(9-a) a polynucleotide comprising the nucleotide sequence of SEQ ID NO: 9;
(9-b) a polynucleotide that hybridizes with a polynucleotide comprising the nucleotide sequence complementary to the nucleotide sequence of SEQ ID NO: 9 under stringent conditions and encodes a protein having the same activity as a protein encoded by the polynucleotide of (9-a);
(9-c) a polynucleotide encoding a protein comprising the amino acid sequence of SEQ ID NO: 10;
(9-d) a polynucleotide encoding a protein comprising an amino acid sequence that is the amino acid sequence of SEQ ID NO: 10 having a deletion, substitution, insertion and/or addition of one or more amino acids, and having the same activity as a protein encoded by the polynucleotide of (9-c); and
(9-e) a polynucleotide encoding a protein having an amino acid sequence with at least 90% identity with respect to the amino acid sequence of SEQ ID NO: 10 and having the same activity as a protein encoded by the polynucleotide of (9-c).

16. A vector according to claim 15, wherein *Arabidopsis thaliana* HSPRO gene untranslated region (5'-UTR) (SEQ ID NO: 18) is attached to the flavone CGT gene.

17. A transgenic plant comprising a polynucleotide according to any one of claims 1 to 3, or an inbred or outbred progeny of the same.

18. The transgenic plant according to claim 17, or its inbred or outbred progeny, wherein the plant is selected from a rose, petunia, chrysanthemum, carnation or lily.

19. The transgenic plant according to claim 18, or its inbred or outbred progeny, wherein the plant is a rose.

20. Propagules, partial plant bodies, tissue or cells of a transgenic plant according to any one of claims 17 to 19, or its inbred or outbred progeny.

21. Cut flowers of a transgenic plant according to any one of claims 17 to 19, or its inbred or outbred progeny, or a processed form created from the cut flowers.

22. A method for creating a transgenic plant with modified flower color, wherein the method comprises a step of causing a delphinidin-type anthocyanin and a flavone C-glycoside to coexist in plant cells, where the 7-position and 4'-position hydroxyl groups of flavone C-glycoside are methylated.

23. The method according to claim 22, wherein the flavone C-glycoside is embigenin.

24. The method according to claim 22 or 23, wherein the delphinidin-type anthocyanin is selected from the group consisting of malvidin 3,5-diglucoside, delphinidin 3,5-diglucoside, petunidin 3,5-diglucoside, acylated delphin and acylated malvin, and their combinations.

25. The method according to any one of claims 22 to 24, comprising a step of introducing a vector according to any one of claims 5 to 16 into plant cells.

26. The method according to claim 25, wherein the plant is a rose, petunia, chrysanthemum, carnation or lily.

27. The method according to claim 26, wherein the plant is a rose.
